(19) Europäisches Patentamt
European Patent Office
Office européen des brevets

(11) EP 3 238 711 B1

(12) EUROPEAN PATENT SPECIFICATION

(45) Date of publication and mention
of the grant of the patent:
**12.07.2023 Bulletin 2023/28**

(21) Application number: **17167718.0**

(22) Date of filing: **24.04.2017**

(51) International Patent Classification (IPC):
**A61K 9/14** (2006.01)    **A61K 47/69** (2017.01)

(52) Cooperative Patent Classification (CPC):
**A61K 9/146**

(54) **BASE FOR SOLID DISPERSION, PRODUCTION METHOD FOR SOLID DISPERSION USING SAME, AND SOLID DISPERSION**

BASIS FÜR FESTE DISPERSION, HERSTELLUNGSVERFAHREN FÜR FESTE DISPERSION DAMIT UND FESTE DISPERSION

BASE POUR DISPERSION SOLIDE, PROCÉDÉ DE PRODUCTION D'UNE DISPERSION SOLIDE L'UTILISANT ET DISPERSION SOLIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2016 JP 2016087648**
**04.04.2017 JP 2017074427**

(43) Date of publication of application:
**01.11.2017 Bulletin 2017/44**

(73) Proprietor: **Mitsubishi Chemical Corporation**
**Tokyo 1008251 (JP)**

(72) Inventors:
• **TSUJI, Kazutoshi**
**Tokyo, 1008251 (JP)**
• **MANDAI, Shusaku**
**Tokyo, 1008251 (JP)**
• **HARA, Kouji**
**Tokyo, 1008251 (JP)**

(74) Representative: **Kuhnen & Wacker**
**Patent- und Rechtsanwaltsbüro PartG mbB**
**Prinz-Ludwig-Straße 40A**
**85354 Freising (DE)**

(56) References cited:
**EP-A1- 2 140 883     US-A- 3 607 812**

• **LAFOUNTAINE JUSTIN S ET AL: "Enabling thermal processing of ritonavir-polyvinyl alcohol amorphous solid dispersions by KinetiSol Dispersing", EUROPEAN JOURNAL OF PHARMACEUTICS AND BIOPHARMACEUTICS, vol. 101, 6 February 2016 (2016-02-06), pages 72-81, XP029459763, ISSN: 0939-6411, DOI: 10.1016/J.EJPB.2016.01.018**

**EP 3 238 711 B1**

**Description**

[Technical Field]

The present invention relates to a use of a base for solid dispersion comprising a polyvinyl alcohol-based resin (hereinafter, abbreviated to "PVA-based resin" in some cases), more particularly, to a use of a base for dispersing a solid-state drug. The present invention further relates to a method for producing solid dispersion comprising the base and to solid dispersion comprising said base. The present invention further relates to a base for solid dispersion for use in a method of treatment of a living body including increasing the amount of active ingredients which are absorbable by the living body.

[Background Art]

When designing medical preparations for oral administration, design of medical preparations to have sufficiently high bioavailability is regarded as important from the standpoints of effectiveness and safety.
One of the important factors which affect the bioavailability of medicines is drug solubility, and a large number of investigations have been made so far on relationships between the solubility and absorption in the digestive tract. Especially in the case of poorly-soluble drugs, it is known that the dissolution rate is a rate determining step of the absorption. Although there are various known medicine preparation methods for improving the solubility of poorly-soluble drugs, solid dispersion especially attract attention.
The solid dispersion is dispersion in which a poorly-soluble drug is dispersed, in a solid state, in a base (inert carrier) for solid dispersion. A method for producing the solid dispersion includes a solvent method, a melting method, a mixing/pulverization method (mechanochemical method), and the like.
The solvent method is a method in which solid dispersion is produced by dissolving both a drug and a base for solid dispersion, as a carrier, in an organic solvent in which the drug and the base are both soluble and then removing the solvent, or by dissolving a drug in an organic solvent, dispersing this solution in a base, and then removing the solvent.
The melting method is a method which utilizes the melting point depression of a drug and a base for solid dispersion. Examples thereof include a method in which both are melted by heating and this is cooled, solidified, and pulverized to obtain solid dispersion; and a method in which a drug is dissolved, with heating, in a water-soluble polymer having a relatively low melting point and this is cooled, solidified, and pulverized to obtain solid dispersion.
The mixing/pulverization method is a method in which a poorly-soluble drug and a base for solid dispersion are subjected, without heating, to mixing/pulverization with a ball mill or to mixing/pulverization by roll mixing or the like, thereby making the poorly-soluble drug to be amorphous to produce solid dispersion.
Mechanochemical is a phenomenon in which mechanical energy (compression, shearing, or friction) causes a change to the physicochemical properties of a substance. In this method, various factors such as lattice defects, lattice irregularities, an increase in specific surface area or surface energy and the like that are caused by a mechanical operation are considered to improve the activity of the solid and to not only render the drug more apt to become amorphous but also make the drug which has become amorphous to be readily dispersed in the base.
Bases for solid dispersion have hitherto been investigated, which uses a compound having a specific solubility in water and an ionic or nonionic polymer (see, for example, Patent Document 1).
Especially in recent years, use of a PVA-based resin is investigated in the expectation that a drug is made to have enhanced amorphous-part stability because of the high glass transition temperature of the PVA-based resin. For example, a base for solid dispersion comprising a PVA-based copolymer has been proposed (see, for example, Patent Document 2).
Patent Document 3 discloses a polyvinyl alcohol resin, Patent Document 4 discloses a base for solid dispersion, Scientific Document 1 discloses the processing of polyvinyl alcohol amorphous solid dispersions utilizing the model compound ritonavir with a specific dispersing technology.

[Prior-Art Documents]

[Patent Documents]

[Patent Document 1] JP2010-536848
[Patent Document 2] WO 2008/133102
[Patent Document 3] US 3 607 812
[Patent Document 4] EP 2 140 883

2

[Scientific Document 1] Justin S. LaFountaine et al: "Enabling thermal processing of ritonavir-polyvinyl alcohol amorphous solid dispersions by KinetiSol Dispersing", European Journal of Pharmaceutics and Biopharmaceutics, Volume 101, 2016, Pages 72-81

[Summary of the Invention]

[Problem that the Invention is to Solve]

However, the bases for solid dispersion disclosed in Patent Documents 1 and 2 are insufficient in terms of drug release rate, and are desired to be further improved.

An object of the present invention is to provide, under such circumstances, a use of a base for solid dispersion which is for obtaining solid dispersion having an excellent drug release rate and less coloration. Further objects of the present invention is to provide a respective solid dispersion and a base for solid dispersion.

[Means for Solving the Problem]

The above-mentioned object is solved by the subject-matter of the independent claims. Advantageous embodiments of the present invention are the subject-matter of the dependent claims.

The present inventors diligently made investigations and, as a result, have found that solid dispersion having an excellent drug release rate can be obtained by using, as a base for solid dispersion, a PVA-based resin containing only a slight amount of a carboxylic acid metal salt. The present invention has been thus completed.

That is, the present invention relates to the use of a base comprising a polyvinyl alcohol-based resin with a carboxylic acid metal salt content of 0.8% by weight or less for solid dispersion, wherein a saponification degree of the polyvinyl alcohol-based resin is 80-95% by mole and an average degree of polymerization of the polyvinyl alcohol-based resin is 200-2,500.

The invention also relates to a method for producing solid dispersion, comprising melt-kneading a mixture of the base for solid dispersion as defined above and a drug, and then cooling the mixture.

The invention also relates to a solid dispersion comprising the base for solid dispersion as defined above.

The invention also relates to a solid dispersion as defined above, further comprising a drug.

The invention also relates to a base for solid dispersion as defined above for use in a method of treatment of a living body including increasing the amount of active ingredients which are absorbable by the living body.

[Effects of the Invention]

According to the present invention, it is possible to obtain a base for solid dispersion which is for obtaining solid dispersion having a high drug release rate. The solid dispersion obtained by using the base for solid dispersion has advantages in that the amount of active ingredients which are absorbable by the living body increases and that the solid dispersion has less coloration. This base is hence highly promising.

The most characteristic feature of the present invention resides in the use of a PVA-based resin which contains only a slight amount of a carboxylic acid metal salt.

Usually, the PVA-based resins available on the market are ones in which the content of a carboxylic acid metal salt (generally sodium acetate) is higher than 1% by weight. Such carboxylic acid metal salts are by-products yielded during the production of the PVA-based resins. However, since the inclusion of the salts in the PVA-based resins has the merits of stabilizing the melt viscosity and obtaining an antiseptic effect, the content of the carboxylic acid metal salts has not been reduced to an unnecessarily low level. The present inventors nevertheless have discovered that a PVA-based resin can be a base for solid dispersion which makes it possible to obtain solid dispersion with less coloration and improved drug release rate, as in the object of the present invention, without impairing the merits.

[Modes for Carrying Out the Invention]

The base for solid dispersion of the present invention is explained below in detail.

The base for solid dispersion of the present invention comprises a PVA-based resin in which a carboxylic acid metal salt content is 0.8% by weight of less.

<PVA-based Resin>

First, the PVA-based resin is explained.

The PVA-based resin is a resin consisting mainly of a vinyl alcohol structural unit which is obtained by saponifying a polyvinyl ester-based resin obtained by polymerizing a vinyl ester-based monomer. The PVA-based resin is composed of vinyl alcohol structural units corresponding to the saponification degree and vinyl ester structural units.

In the PVA-based resin to be used in the present invention, a carboxylic acid metal salt content is 0.8% by weight or less.

Examples of carboxylic acid of the carboxylic acid metal salt include formic acid, acetic acid, propionic acid, butyric acid, valeric acid, caproic acid, enanthic acid, caprylic acid, pelargonic acid, capric acid, lauric acid, myristic acid, palmitic acid, margaric acid, stearic acid, oleic acid, sorbic acid, oxalic acid, malonic acid, succinic acid, glutaric acid, adipic acid, fumaric acid, and maleic acid. Preferred of these, from the standpoint of solubility in water, are formic acid, acetic acid, propionic acid, and caprylic acid. Acetic acid is especially preferred.

Examples of the metal salt include alkali metal salts such as sodium and potassium and divalent metal salts such as magnesium and calcium. Of these, alkali metal salts are preferred from the standpoint that the effect thereof is high for the use amount.
Especially preferred are sodium salts.

Specifically, sodium acetate is the most preferred.

The base for solid dispersion of the present invention is one in which the PVA-based resin contains the carboxylic acid metal salt in an amount of 0.8% by weight or less, preferably 0.4% by weight or less, especially preferably 0.2% by weight or less. In case where the content is too high, the effects of the present invention are difficult to obtain. Meanwhile, from the standpoint of the stability of the melt viscosity, it is preferable that a lower limit of the content should be 0.01% by weight, in particular 0.02% by weight, especially 0.03% by weight.

With respect to methods for incorporating a carboxylic acid metal salt, PVA-based resins usually contain a carboxylic acid metal salt, such as sodium acetate, yielded as a byproduct of saponification, in an amount of about 1.0-2.0% by weight. Consequently, a preferred method for regulating the content to 0.8% by weight or less is to clean the resin with an alcohol-based organic solvent such as methanol.

The saponification degree of the PVA-based resin to be used in the present invention is 80-95% by mole, preferably 83-93% by mole, more preferably 85-90% by mole. Too low or too high saponification degree of the PVA-based resin tends to result in a decrease in drug release rate.

In the present invention, the saponification degree of PVA-based resin is a value determined by a method according to JIS K 6726.

The average degree of polymerization of the PVA-based resin to be used in the present invention is 200-2,500, preferably 400-1,500, especially preferably 300-1,000.

In case where the average degree of polymerization of the PVA-based resin is too low, the resin tends to have reduced strength and be brittle. In case where the average degree of polymerization is too high, the resin tends to have an elevated melting point, making it difficult to produce solid dispersion by the melting method.

In the present invention, the average degree of polymerization of polyvinyl alcohol is an average degree of polymerization determined by a method according to JIS K 6726.

A method for producing the PVA-based resin to be used in the present invention is explained here in more detail.

The PVA-based resin is obtained, for example, by saponifying a polyvinyl ester-based polymer obtained by polymerizing a vinyl ester-based monomer. Examples of the vinyl ester-based monomer include vinyl formate, vinyl acetate, vinyl propionate, vinyl valerate, vinyl butyrate, vinyl isobutyrate, vinyl pivalate, vinyl caprate, vinyl laurate, vinyl stearate, vinyl benzoate, and vinyl versatate. Vinyl acetate is suitable from the standpoint of profitability.

Monomers having copolymerizability with these vinyl ester-based monomers can be copolymerized so long as this copolymerization does not lessen the effects of the present invention. Examples of such comonomers include: olefins such as ethylene, propylene, isobutylene, $\alpha$-octene, $\alpha$-dodecene, and $\alpha$-octadecene; hydroxyl-containing $\alpha$-olefins such as 3-buten-1-ol, 4-penten-1-ol, 5-hexen-1-ol, and 3,4-dihydroxy-1-butene and derivatives thereof such as acylated product; unsaturated acids such as acrylic acid, methacrylic acid, crotonic acid, maleic acid, maleic anhydride, itaconic acid, and undecylenic acid, and salts thereof; and esters thereof such as monoester or dialkyl ester; nitriles such as acrylonitrile and methacrylonitrile; amides such as diacetoneacrylamide, acrylamide, and methacrylamide; olefinsulfonic acids such as ethylenesulfonic acid, allylsulfonic acid, and methallylsulfonic acid and salts thereof; vinyl compounds such as alkyl vinyl ethers, dimethylallyl vinyl ketone, N-vinylpyrrolidone, vinyl chloride, vinylethylene carbonate, 2,2-dialkyl-4-vinyl-1,3-dioxolanes, and glycerin monoallyl ether; substituted vinyl acetates such as isopropenyl acetate and 1-methoxyvinyl acetate; and vinylidene chloride, 1,4-diacetoxy-2-butene, 1,4-dihydroxy-2-butene, vinylene carbonate, 1,3-diacetoxy-2-methylenepropane, 1,3-dipropionyloxy-2-methylenepropane, and hydroxymethylvinylidene diacetate such as 1,3-dibutyronyloxy-2-methylenepropane. The content of such comonomers is usually 10% by mole or less, preferably 5% by mole or less, especially preferably 1% by mole or less, based on the whole polymer. In the present invention, an unmodified PVA consisting only of vinyl-alcohol structural units and vinyl-ester structural units, which are unsaponified portions, is preferred from the standpoint of safety.

When polymerizing the vinyl ester-based monomer and a comonomer, a known method such as bulk polymerization, solution polymerization, suspension polymerization, dispersion polymerization, or emulsion polymerization can be employed without particular limitations. Usually, however, solution polymerization is conducted.

Examples of solvents usable in the polymerization usually include aliphatic alcohols having 1-4 carbon atoms, such as methanol, ethanol, isopropyl alcohol, n-propanol, and butanol, and ketones such as acetone and methyl ethyl ketone. Industrially, use of methanol is suitable.

The polymerization reaction is conducted using, for example, a known radical polymerization catalyst such as azobisisobutyronitrile, acetyl peroxide, benzoyl peroxide, or lauroyl peroxide or any of various known low-temperature active catalysts. A reaction temperature is selected from the range of from 35°C to the boiling point of the catalyst selected from known radical polymerization catalysts and various known low-temperature active catalysts.

The polyvinyl ester-based polymer obtained is subsequently saponified continuously or batchwise. Although either alkali saponification or acid saponification can be employed for the saponification, alkali saponification is industrially suitable in which the polymer is dissolved in an alcohol and saponified in the presence of an alkali catalyst.

Preferred as the alcohol is an aliphatic alcohol having 1-4 carbon atoms, such as methanol, ethanol, isopropyl alcohol, n-propanol, or butanol. More preferred of these are methanol and ethanol. The concentration of the polymer in the alcohol is selected from the range of 20-60% by weight. Water may be added according to need in an amount of about 0.3-10% by weight. Further, various esters, such as methyl acetate, and various solvents including benzene, hexane, and DMSO (dimethyl sulfoxide) may be added.

Examples of the alkali catalyst include alkali catalysts such as the hydroxides and alcoholates of alkali metals, e.g., sodium hydroxide, potassium hydroxide, sodium methylate, sodium ethylate, and potassium methylate. It is preferable that the amount of the catalyst to be used should be 1-100 mmol-equivalent to the monomers.

After the saponification, the PVA-based resin obtained is cleaned with a cleaning liquid. Examples of the cleaning liquid include alcohols such as methanol, ethanol, isopropyl alcohol, and butanol. Methanol is preferred from the standpoints of cleaning efficiency and drying efficiency.

Examples of methods for the cleaning include a batchwise cleaning method and a continuous cleaning method. Usually, a batchwise cleaning method is employed. Examples of stirring modes (devices) for the cleaning include screw blades, ribbon blenders, and kneaders.

Examples of cleaning devices include cylindrical cleaning devices, countercurrent contact type cleaning devices, and centrifugal cleaning devices.

The bath ratio [(mass of the cleaning liquid) / (mass of the polyvinyl ester-based polymer particles)] is usually 1-30, especially preferably 2-20. In case where the bath ratio is too high, a large cleaning device tends to be necessary, resulting in an increase in cost. In case where the bath ratio is too low, the cleaning effect tends to be low, resulting in an increase in the number of cleaning operations.

The temperature during the cleaning is usually 10-80°C, especially preferably 20-70°C. In case where the temperature is too high, the cleaning liquid tends to volatilize in an increased amount, resulting in the necessity of a reflux device. In case where the temperature is too low, the cleaning efficiency tends to be low. The cleaning time is usually 5 minutes to 12 hours, especially preferably 30 minutes to 4 hours. In case where the cleaning time is too long, the production efficiency tends to decrease. In case where the cleaning time is too short, the cleaning tends to be insufficient and the carboxylic acid metal salt tends to remain in a large amount. The number of cleaning operations is usually 1-10, especially preferably 1-5. In case where the number of cleaning operations is too large, a decrease in production efficiency and an increase in cost tend to result.

The cleaned particles of the saponified polyvinyl ester-based polymer are dried with, for example, hot air either continuously or batchwise, thereby obtaining a powder of a PVA-based resin for use in the present invention. The drying temperature is usually 50-150°C, particularly preferably 60-130°C, especially preferably 70-110°C. In case where the drying temperature is too high, the PVA-based resin tends to deteriorate thermally. In case where the drying temperature is too low, the drying tends to require a prolonged time period. The drying time is usually 1-48 hours, especially preferably 2-36 hours. In case where the drying time is too long, the PVA-based resin tends to deteriorate thermally. In case where the drying time is too short, the drying tends to be insufficient or a higher temperature tends to be required for the drying.

The amount of the solvent contained in the dried powder of the PVA-based resin is usually 0-10% by weight, and is regulated to particularly preferably 0.01-5% by weight, especially preferably 0.1-1% by weight.

Thus, the PVA-based resin for use in the present invention is obtained, and the base for solid dispersion of the present invention is obtained by incorporating this resin.

In the base for solid dispersion of the present invention, the content of the PVA-based resin is preferably 20% by weight or higher, more preferably 30-90% by weight, even more preferably 40-80% by weight, based on the whole base for solid dispersion. In case where the content thereof is too low, the amorphous state of a drug tends to be unable to be maintained. In case where the content of the PVA-based resin is too high, a decrease in drug content results and this undesirably tends to result in an increase in the amount of the drug to be administered in order to produce the effect of the drug.

The solid dispersion of the present invention includes the base for solid dispersion of the present invention and a drug.

<Drug>

The drug is explained next.

Examples of the drug to be contained in the solid dispersion of the present invention include the following.

(1) Antipyretic, analgetic, and anti-inflammatory agents

Examples include salicylic acid, sulpyrine, flufenamic acid, diclofenac, indomethacin, atropine, scopolamine, morphine, pethidine, levorphanol, ketoprofen, naproxen, ibuprofen, oxymorphone, aspirin, aminopyrine, phenacetin, acetoaminophenone, phenylbutazone, ketophenylbutazone, mefenamic acid, bucolome, benzydamine, mepirizole, tiaramide, tinoridine, xylocaine, pentazocine, dexamethasone, hydrocortisone, prednisolone, azulene, isopropylantipyrine, sasapyrine, clofezone, etodolac, and salts of these.

(2) Tranquilizing agents

Examples include diazepam, lorazepam, oxazepam, oxazolam, clotiazepam, medazepam, temazepam, fludiazepam, meprobamate, nitrazepam, and chlorodiazepoxide.

(3) Antipsychotic agents

Examples include chlorpromazine, prochlorperazine, trifluoperazine, sulpiride, clocapramine hydrochloride, zotepine, and haloperidol.

(4) Antimicrobial agents

Examples include griseofulvin, Lankacidin species [J. Antibiotics, 38, 877-885 (1985)], azole compounds (such as 2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl-3-(2H,4H)-1,2,4-triazolon, fluconazole, and itraconazole), nalidixic acid, piromidic acid, pipemidic acid trihydrate, enoxacin, cinoxacin, ofloxacin, norfloxacin, ciproxacin hydrochloride, sulfamethoxazole, and trimethoprim.

(5) Antibiotic agents

Examples include gentamicin, dipekacin, kanendomycin, lividomycin, topramycin, amikacin, dibekacin, fradiomycin, sisomicin, tetracycline, oxytetracycline, rolitetracycline, doxycycline, ampicillin, piperacillin, ticarcillin, cephalothin, cephaloridine, cefotiam, cefotiam hexetil, cefsulodin, cefmenoxime, cefmetazole, cefazolin, cefotaxime, cefoperazone, ceftizoxime, moxalactam, thienamycin, sulfazecin, azthreonam, amoxicillin, cefalexin, erythromycin, bacampicin, minocycline, chloramphenicol, and salts of these.

(6) Antineoplastic agents

Examples include 6-O-(N-chloroacetylcarbamoyl)fumagillol, bleomycin, methotrexate, actinomycin D, mitomycin C, daunorubicin, adriamycin, neocarzinostatin, cytosine arazinoside, fluorouracil, tetrahydrofuryl-5-fluorouracil, picibanil, lentinan, levamisole, bestatin, adimexon, glycyrrhizin, HER2 inhibitors (the heterocyclic compounds, etc. given in WO 01/77107 etc.), taxol, doxorubicin hydrochloride, etoposide, mitoxantrone, mesna, dimesna, aminoglutethimide, tamoxifen, acrolein, cisplatin, carboplatin, cyclophosphamide, lomustine (CCNU), and carmustine (BCNU).

(7) Antihyperlipidemic agents

Examples include clofibrate, ethyl 2-chloro-3-[4-(2-methyl-2-phenylpropoxy)phenyl]propionate [Chem. Pharm. Bull., 38, 2792-2796 (1990)], clinofibrate, colestyramine, soysterol, tocopherol nicotinate, nicomol, niceritrol, probucol, and elastase.

(8) Antitussive/expectorant agents

Examples include ephedrine, methylephedrine, noscapine, codeine, dihydrocodeine, alloclamide, chlorphedianol, picoperidamine, cloperastine, protokylol, isoproterenol, salbutamol, terputaline, bromhexine, carbocisteine, ethylcisteine, methylcisteine, and salts of these.

(9) Muscle relaxant agents

Examples include pridinol, tubocurarine, pancuronium, chlorphenesin carbamate, tolperisone hydrochloride, eperisone hydrochloride, tizanidine hydrochloride, mephenesin, chlorzoxazone, phenprobamate, methocarbamol, chlormezanone, pridinol mesilate, afloqualone, baclofen, and dantrolene sodium.

(10) Antiepileptic agents

Examples include phenytoin, ethosuximide, acetazolamide, chlordiazepoxide, phenobarbital, carbamazepine, and primidone.

(11) Antiulcer agents

Examples include lansoprazole, metoclopramide, famotidine, omeprazole, sulpiride, trepibutone, cetraxate hydrochloride, gefarnate, irsogladine maleate, cimetidine, ranitidine hydrochloride, nizatidine, and roxatidine acetate hydrochloride.

(12) Antidepressant agents

Examples include imipramine, clomipramine, noxiptilline, and phenelzine.

(13) Antiallergic agents

Examples include diphenhydramine, chlorpheniramine, tripelennamine, metodilamine, clemizole, diphenylpyraline, methoxyphenamine, clemastine fumarate, cyproheptadine hydrochloride, mequitazine, and alimemazine tartrate.

(14) Cardiotonic agents

Examples include trans-bioxocamphor, terephyllol, aminophylline, and etilefrine.

(15) Anti-arrhythmic agents

Examples include propranol, alprenolol, pufetolol, oxprenolol, procainamide hydrochloride, disopyramide, ajmaline, quinidine sulfate, aprindine hydrochloride, propafenone hydrochloride, and mexiletine hydrochloride.

(16) Vasodilators

Examples include oxyfedrine, diltiazem, tolazoline, hexobendine, bamethane, nifedipine, nilvadipine, isosorbide dinitrate, diltiazem hydrochloride, trapidil, dipyridamole, dilazep hydrochloride, verapamil, nicardipine hydrochloride, ifenprodil tartrate, cinepacide maleate, ciclandelate, cynnaridine, and pentoxyphylin.

(17) Hypotensive diuretic agents

Examples include hexamethonium bromide, pentolinium, mecamylamine, ecarazine, clonidine, diltiazem, nifedipine, furosemide, trichlormethiazide, methychlothiazide, hydrochlorothiazide, hydroflumethiazide, ethiazide, cyclopenthiazide, fluorothiazide, and ethacrynic acid.

(18) Anti-diabetic agents

Examples include glymidine, glipuzide, phenformin, puformin, metformin, glibenclamide, and tolbutamide.

(19) Antitubercular agents

Examples include isoniazid, ethambutol, and para-aminosalicylic acid.

(20) Antinarcotic agents

Examples include levallorphan, nalorphine, naloxone, and salts of these.

(21) Hormone agents

Examples include steroid hormones such as dexamethasone, hexestrol, methimazole, petamethasone, triamcinolone, triamcinolone acetonide, fluocinolone acetonide, prednisolone, hydrocortisone, and estriol.

(22) Prophylactic/therapeutic agents for bone/cartilage diseases

Examples include non-peptidic substances functioning to promote bone formation such as prostaglandin A1 derivatives, vitamin D derivatives, vitamin $K_2$ derivatives, eicosapentaenoic acid derivatives, benzylphosphonic acid, bisphosphonic acid derivatives, sex hormone derivatives, phenolsulfophthalein derivatives, benzothiopyran or benzothiepin derivatives, thienoindazole derivatives, menatetrenone derivatives, and helioxanthin derivatives, and peptidic substances promoting bone formation.

(23) Joint disease therapeutics

Examples include p-38 MAP kinase inhibitors (the thiazole compounds, etc. given in WO 00/64894, etc.), matrix metalloproteinase inhibitors (MMPI), anti-inflammatory steroids such as prednisolone, hydrocortisone, methylprednisolone, dexabetamethasone, and betamethasone, and non-steroidal antiphlogistics such as indomethacin, diclofenac, loxoprofen, ibuprofen, piroxicam, and sulindac.

(24) Frequent urination therapeutic hydrochlorides

Examples include flavoxate, oxybutynin hydrochloride, and terolysine hydrochloride.

(25) Anti-androgen agents

Examples include oxendolone, allylestrenol, chlormadinone acetate, gestonorone caproate, osapron acetate, flutamide, and bicalutamide.

(26) Fat-soluble vitamin agents

Examples include vitamin K's such as vitamins $K_1$, $K_2$, $K_3$, and $K_4$ and folic acid (vitamin M).

(27) Vitamin derivatives

Examples include various vitamin derivatives such as vitamin $D_3$ derivatives including 5,6-trans-cholecalciferol, 2,5-hydroxycholecalciferol, and 1-$\alpha$-hydroxycholecalciferol and vitamin $D_2$ derivatives including 5,6-trans-ergocalciferol.

(28) Others

Examples include hydroxycam, diacerin, megestrol acetate, nicergoline, and prostaglandins, and further include ischemic disease therapeutics, immune disease therapeutics, Alzheimer's disease therapeutics, osteoporosis therapeutics, angiogenesis therapeutics, retinopathy therapeutics, retinal vein occlusion therapeutics, senile disciform macular degeneration therapeutics, cerebral vasospasm therapeutics, cerebral thrombosis therapeutics, brain infarction therapeutics, cerebral embolism therapeutics, intracerebral bleeding therapeutics, subarachnoidal bleeding therapeutics, hypertensive encephalopathy therapeutics, transient cerebral ischemic attack therapeutics, multi infarct dementia therapeutics, arteriosclerosis therapeutics, Huntington's disease therapeutics, cerebral tissue dysfunction therapeutics, optic nerve disease therapeutics, glaucoma therapeutics, ocular hypertension therapeutics, retinal detachment therapeutics, arthritis therapeutics, antirheumatic drugs, antisepsis drugs, antiseptic shock drugs, antiasthmatic drugs, atopic dermatitis therapeutics, and allergic rhinitis therapeutics.

From the standpoint of solubility improvement, poorly-soluble drugs are preferred. Examples of such poorly-soluble drugs include carbamazepine, indomethacin, naproxen, ibuprofen, phenacetin, phenylbutazone, griseofulvin, the azole compounds, phenytoin, isosorbide dinitrate, and nitrophenylpyridine compounds. The nitrophenylpyridine compounds include poorly-soluble compounds each having a nitrophenyl group and a pyridine ring structure. Preferred nitrophenylpyridine compounds are compounds having a structure in which a nitrophenyl group has been bonded to any of the 2- to 4-positions in the pyridine ring. Specific compounds include nifedipine and nilvadipine.

The content of the drug is suitably regulated in accordance with bioavailability. The drug may be one which has been diluted with, for example, a diluent for general use in the clinical field or in the fields of foods, etc. Use may also be made of a drug which has been treated in order to mask the bitterness thereof.

<Other Additives>

Various additives can be added to the solid dispersion of the present invention so long as the addition thereof does not lessen the effects of the present invention. Examples of the additives include an excipient, disintegrator, pH regulator, fluidizing agent, surfactant, colorant, sweetener, and coating material.

As the excipient, for example, one or more ingredients selected from sugar alcohols, saccharides, calcium phosphates, crystalline celluloses, starches, sodium phosphates, gelatins, and the like are used. Preferred excipients include sugar alcohols and saccharides.

Examples of the sugar alcohols include mannitol, erythritol, xylitol, sorbitol, and maltitol. Examples of the saccharides include glucose, fruit sugar, lactose, refined sugar, trehalose, maltose, and oligosaccharides.

Examples of the disintegrator include calcium calmellose, sodium carboxymethyl starch, sodium croscarmellose, crospovidone, cellulose or derivatives thereof, and starches or derivatives thereof.

Examples of the pH regulator include citric acid and salts thereof, phosphoric acid and salts thereof, carbonic acid and salts thereof, tartaric acid and salts thereof, fumaric acid and salts thereof, acetic acid and salts thereof, amino acids and salts thereof, succinic acid and salts thereof, and lactic acid and salts thereof.

Examples of the fluidizing agent include light anhydrous silicic acid, hydrous silicon dioxide, titanium oxide, stearic acid, corn gel, and heavy anhydrous silicic acid.

Examples of the surfactant include phospholipids, glycerin fatty acid esters, polyoxyethylene fatty acid esters, sorbitan fatty acid esters, polyethylene glycol fatty acid esters, polyoxyethylene hardened castor oil, polyoxyethylene alkyl ethers, sucrose fatty acid esters, sodium lauryl sulfate, polysorbates, sodium hydrogen phosphates, and potassium hydrogen phosphates.

Examples of the colorant include iron sesquioxide, yellow iron sesquioxide, Food Yellow No. 5, Food Yellow No. 4, aluminum chelates, titanium oxide, and talc.

Examples of the sweetener include saccharin, aspartame, potassium acesulfum, thaumatin, and sucralose.

Examples of the coating material include hydroxypropyl cellulose, hydroxypropyl methyl cellulose, methyl cellulose, polyvinyl alcohol, polyvinylpyrrolidone, a dispersion of an ethyl acrylate/methyl methacrylate copolymer, hydroxypropyl methyl cellulose acetate succinate, and methacrylic acid copolymers.

<Processes for producing the Solid Dispersion>

Examples of a method for producing the solid dispersion of the present invention include the following:

(i) Solvent method: a method in which a drug and a base are dissolved in an organic solvent and the solvent is removed thereafter.
(ii) Melting method: a method in which a drug and a base are melt-kneaded with heating and the mixture is then cooled and solidified.
(iii) Mixing/pulverization method (mechanochemical method): a method in which a drug is reduced into finer particles or is dispersed in a base, using mechanical energy such as compression, shearing, or friction.

Among these methods, (ii) the melting method is preferred from the standpoint of controlling the crystals.

The melting method is described below in detail.

The melting method is a method in which a mixture of a drug and a base for solid dispersion is heat-treated and the heat-treated mixture is subjected, according to need, to shape regulation (pulverization, powdering, molding, etc.), thereby obtaining solid dispersion.

The heat treatment of the mixture of a drug and the base for solid dispersion of the present invention may be conducted at a temperature at which the mixture is wholly melted, or at a temperature at which some of the two ingredients are melted. It is possible to select temperature conditions under which some or all of the drug becomes amorphous depending on the properties thereof even when melting is not reached.

Solid dispersion can be obtained even when melting is not reached, by making some or all of the drug amorphous, and this case is also included in the melting method in the present invention.

The term "mixture of a drug and a base for solid dispersion" means any of mixtures such as a mixture obtained by incorporating a drug into a molten base for solid dispersion and a mixture obtained by dry-blending a base for solid dispersion with a drug and then melt-kneading the blend.

Heating means usable for the heat treatment are not particularly limited, and suitable heating means usable by persons skilled in the art can be employed. More specifically, examples thereof include heating with a drying oven, oil bath, electric furnace, and the like, heating by ultrasonic wave propagation, heating by treatment with twin-screw kneading, heating by treatment with single-screw and twin-screw extrusion (extruder), and heating by irradiation with microwaves.

Means for regulating the shape of the heat-treated mixture are not particularly limited, and suitable means usable by persons skilled in the art can be employed. For example, pulverization means, molding means, and the like can be employed. Examples of the molding means include extrusion molding (e.g., single-screw extrusion, twin-screw extrusion, multi-screw extrusion, and hot-melt calendering), injection molding (e.g., twin-screw extruder), and compression molding (e.g., tableting and granulation).

In the case where production of solid dispersion by a heat treatment and shape regulation of the solid dispersion is simultaneously conducted, a device which is for regulating the shape of heat-treated mixtures and which is equipped with a heating means can be used.

The heat treatment need not be always performed at a temperature at which the mixture being heated is melted. It is known that solid dispersion can be produced at a heating temperature lower than the melting temperatures. In the present invention also, solid dispersion is produced at a temperature as low as possible. Thus, even a drug which suffers decomposition, deterioration, etc. at high temperatures can be used to produce solid dispersion thereof. The heat-treatment temperature is usually 80-220°C, preferably 120-200°C, more preferably 150-200°C. Incidentally, the ultrasonic wave propagation is a treatment in which ultrasonic waves are propagated to the powder mixture. In the case of the heating by ultrasonic wave propagation, the ultrasonic wave propagation energy is usually 600-2,000 J, preferably 700-1,800 J, more preferably 800-1,300 J. As a device for such treatment, use can be made, for example, of ultrasonic-wave molding machine USTM/L20, manufactured by Tecnea Engineering. When using this device, a powder mixture including a drug and a base for powder dispersions and optionally containing additives is charged into the mortar provided to the device and the mixture is compression-molded while propagating ultrasonic waves thereto.

There are twin-screw extrusion molding machines, twin-screw extruders, and the like which are equipped with heating means. It is preferable that the heat treatment in the present invention should be one in which solid dispersion is produced by a means for ultrasonic wave propagation and simultaneous compression molding, or with a twin-screw extrusion molding machine equipped with a heating means, or with a twin-screw extruder equipped with a heating means.

Thus, solid dispersion comprising the base for solid dispersion of the present invention can be obtained. The solid dispersion obtained has an excellent drug release rate and is reduced in coloration.

[Examples]

The present invention will be explained below in more detail by reference to Examples. In the following Examples and Comparative Example, "parts" and "%" are by weight.

Example 1 [Production of base for solid dispersion]

Into a reaction tank equipped with a reflux condenser, dropping funnel, and stirrer were introduced 280 parts of vinyl acetate, 300 parts of methanol, and 0.10% by mole azobisisobutyronitrile (based on the vinyl acetate introduced). While stirring the contents in a nitrogen stream, the temperature of the contents was elevated until refluxing began and, at 30 minutes after the initiation of refluxing, 720 parts of vinyl acetate was dropped at a constant rate over 9.5 hours to thereby conduct polymerization. After completion of the dropwise addition and at the time when the conversion into polymer of the vinyl acetate had reached 92%, m-dinitrobenzene was added to terminate the polymerization. Subsequently, the unreacted vinyl acetate monomer was removed from the system by bubbling methanol vapor into the mixture. Thus, a methanol solution of a vinyl acetate polymer was obtained (resin content, 53%).

Subsequently, the methanol solution was further diluted with methanol to adjust the concentration thereof to 50%, and the diluted solution was introduced into a kneader. While keeping the solution temperature at 35°C, a 4% methanol solution of sodium hydroxide was added thereto in such a proportion that the amount of sodium hydroxide was 6.0 mmol per mole of vinyl-acetate structural units contained in the polymer, thereby performing saponification. As the saponification proceeded, a saponification product precipitated. At the time when the product had become particulate, this product was taken out by solid-liquid separation by filtration. This saponification product was dried.

The saponification degree of the dried PVA-based resin powder obtained was determined by analyzing the powder for alkali consumption required for hydrolyzing the residual vinyl acetate. As a result, the saponification degree thereof was found to be 88% by mole. A 4% aqueous solution of the powder had a viscosity of 5.6 mPa·s. The average degree of polymerization of the powder was 600.

The dried PVA-based resin powder obtained was added to methanol in a bath ratio of 10, and the mixture was stirred for 3 hours to clean the powder. Thereafter, the mixture was subjected to solid-liquid separation and the PVA-based resin powder obtained was vacuum-dried at 90°C until the volatile content decreased to 1% or less. Thus, a dry powder of the PVA-based resin was obtained. This powder was used as a base for solid dispersion.

The content of sodium acetate was determined by dissolving the dry powder of the PVA-based resin in water and subjecting the solution to neutralization titration with hydrochloric acid using Methyl Orange as an indicator. The content of sodium acetate was found to be 0.1%.

The method for the neutralization titration is explained below in detail.

First, 3.00 g of the dry powder of the PVA-based resin and 100 mL of water were introduced into an Erlenmeyer flask, and this flask was placed on a device for dissolution by heating and stirring. The heating and stirring was continued until the dry powder of the PVA-based resin was completely dissolved. Next, the air-cooled tube was removed, and the flask was allowed to stand for 30 minutes to remove any solvent remaining in the dry powder of the PVA-based resin and to cool the solution to room temperature.

Three drops of Methyl Orange were added to each of 100 mL of the aqueous PVA-based-resin solution obtained and 100 mL of pure water (blank test; reference color). 0.1-N HCl was continuously added drop by drop to the aqueous PVA-based-resin solution until this solution assumed the same color as the pure water. The amount of the sodium acetate was calculated from the amount of the 0.1-N HCl required for the neutralization.

[Production of solid dispersion]

Forty-four parts of the base for solid dispersion and 11 parts of carbamazepine as a drug were mixed together, and this mixture was melt-kneaded with a twin-screw kneading machine (Plastograph, manufactured by Brabender GmbH & Co.) under the following conditions. After completion of the kneading, the melt-kneaded mixture was taken out from the inside and cooled at room temperature. Thus, solid dispersion of the present invention was obtained.

(Melt kneading conditions)

| | |
|---|---|
| Screw rotation speed: | 50 rpm |
| Set temperature : | 200°C |
| Preheating time : | 3 min |
| Kneading time : | 5 min |

The solid dispersion obtained was subjected to the following evaluation.

[Evaluation of release rate]

The release rate was determined by the dissolution test, second method according to the Japanese Pharmaceutical Codex, third volume "Carbamazepine Fine Granules". In order to calculate the release rate, the absorbance was measured with a UV absorptiometer (spectrophotometer for ultraviolet and visible regions "V-560", manufactured by Shimadzu Corp.). Specifically, 900 mL of water was introduced into a glass vessel equipped with stirring blades, and 1.2 g of the solid dispersion obtained above was introduced thereinto. Thereafter, the contents were stirred, and 20 mL of a sample was taken out after 180 minutes. This sample was filtered with a membrane filter (DISMIC; 0.45 $\mu$m; diameter, 25 mm; manufactured by ADVANTEC Co., Ltd.), and a 2-mL portion was collected from the filtrate and diluted to 50 mL, thereby obtaining a test sample.

Separately therefrom, known carbamazepine (manufactured by Wako Pure Chemical Industries, Ltd.) having a purity of 98% or higher was dried at 105°C for 2 hours, and an about 0.022-g portion thereof was weighed out and dissolved in 10 mL of methanol. Water was added thereto to adjust the volume thereof precisely to 100 mL. Four milliliters of this liquid was diluted with water to 100 mL to obtain a standard solution. The standard solution, the sample solution, and water as a reference were examined at a wavelength of 285 nm by absorptiometry in ultraviolet and visible regions (spectrophotometer for ultraviolet and visible regions "V-560", manufactured by Shimadzu Corp.) to measure the absorbances At and As.

The release rate of carbamazepine was calculated using the following equation. The results are shown in Table 1.

$$\text{Release rate (\%)} = (\text{Ws/Wt}) \times (\text{At/As}) \times (1/\text{C}) \times 900$$

Ws: collected amount (mg) of the standard carbamazepine product
Wt: collected amount (g) of the sample for release rate measurement
C: amount (mg) of carbamazepine in 1 g of the sample
At: absorbance (abs.) at 285 nm of the sample

As: absorbance (abs.) at 285 nm of the carbamazepine standard solution

[Evaluation of coloration]

The solid dispersion was formed into a plate under the following conditions and examined for YI (yellowness index) to evaluate the coloration. (Plate production method)

Device: single-acting compression molding machine Type NSF-37 (manufactured by Shinto Metal Industries Corp.)
Temperature: 220°C
Preheating: 8 min
Pressing time: 10 min
Plate size: 30 mm × 50 mm × 0.8 mm

(YI measuring conditions)

Device: spectral color-difference meter SE-6000 (manufactured by Nippon Denshoku Kogyo K.K.)
Measuring method: reflection method
Number of measurements: 2

Example 2

The same procedure as in Example 1 was conducted, except that the PVA cleaning bath ratio was changed to 5 and the cleaning time was changed to 1 hour. The resin powder and the solid dispersion were evaluated in the same manners. The results are shown in Table 1.

Comparative Example 1

The same procedure as in Example 1 was conducted, except that the step of cleaning the PVA-based resin was omitted. The resin powder and the solid dispersion were evaluated in the same manners. The results are shown in Table 1.

Table 1

|  | Sodium acetate (%) | Release rate (%) | YI value |
|---|---|---|---|
| Example 1 | 0.1 | 91.2 | 76 |
| Example 2 | 0.5 | 87.6 | 84 |
| Comparative Example 1 | 1 | 83.4 | 128 |

The solid dispersion which employed the base for solid dispersion according to the present invention (Examples 1 and 2) each showed a high release rate and excellent bioavailability. On the other hand, Comparative Example 1, which had a high sodium acetate content, showed a low release rate and low bioavailability.

Furthermore, the solid dispersion which employed the base for solid dispersion according to the present invention each had a small YI value and has less coloration. On the other hand, Comparative Example 1, which had a high sodium acetate content, had high coloration.

[Industrial Applicability]

The solid dispersion of the present invention has a high drug release rate and high bioavailability and has less coloration. This solid dispersion is hence useful for medicalpreparations.

**Claims**

1.   Use of a base comprising a polyvinyl alcohol-based resin with a carboxylic acid metal salt content of 0.8% by weight or less, for solid dispersion, wherein a saponification degree of the polyvinyl alcohol-based resin is 80-95% by mole and an average degree of polymerization of the polyvinyl alcohol-based resin is 200-2,500.

2. A method for producing solid dispersion, comprising melt-kneading a mixture of the base for solid dispersion as defined in claim 1 and a drug, and then cooling the mixture.

3. Solid dispersion comprising the base for solid dispersion as defined in claim 1.

4. Solid dispersion according to claim 3, further comprising a drug.

5. A base for solid dispersion as defined in claim 1 for use in a method of treatment of a living body including increasing the amount of active ingredients which are absorbable by the living body.


**Patentansprüche**

1. Verwendung einer Basis, welche ein Harz auf Polyvinylalkoholbasis mit einem Gehalt an Carbonsäuremetallsalz von 0,8 Gew.-% oder weniger aufweist, für eine feste Dispersion, wobei ein Verseifungsgrad des Harzes auf Polyvinylalkoholbasis 80-95 Mol-% beträgt und ein mittlerer Polymerisationsgrad des Harzes auf Polyvinylalkoholbasis 200-2.500 beträgt.

2. Verfahren zur Herstellung einer festen Dispersion, welches ein Schmelzkneten eines Gemischs aus der Basis für die feste Dispersion nach Anspruch 1 und eines Arzneistoffs und dann Kühlen des Gemischs aufweist.

3. Feste Dispersion, welche die Basis für die feste Dispersion nach Anspruch 1 aufweist.

4. Feste Dispersion nach Anspruch 3, welche ferner einen Arzneistoff aufweist.

5. Basis für die feste Dispersion nach Anspruch 1 zur Verwendung in einem Verfahren zur Behandlung eines lebenden Körpers, welches ein Erhöhen der Menge an Wirkstoffen, die durch den lebenden Körper absorbiert werden können, beinhaltet.


**Revendications**

1. Utilisation d'une base comprenant une résine à base de poly(alcool vinylique) avec une teneur en sel de métal d'acide carboxylique de 0,8 % en masse ou inférieure, pour dispersion solide, dans laquelle un degré de saponification de la résine à base de poly(alcool vinylique) est de 80-95 % en mole et un degré moyen de polymérisation de la résine à base de poly(alcool vinylique) est de 200-2 500.

2. Procédé de production de dispersion solide, comprenant le malaxage à l'état fondu d'un mélange de la base pour dispersion solide selon la revendication 1 et d'un médicament, et ensuite le refroidissement du mélange.

3. Dispersion solide comprenant la base pour dispersion solide selon la revendication 1.

4. Dispersion solide selon la revendication 3, comprenant de plus un médicament.

5. Base pour dispersion solide selon la revendication 1 pour une utilisation dans un procédé de traitement d'un corps vivant incluant l'augmentation de la quantité d'ingrédients actifs qui peuvent être absorbés par le corps vivant.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010536848 A
- WO 2008133102 A
- US 3607812 A
- EP 2140883 A
- WO 0177107 A
- WO 0064894 A

**Non-patent literature cited in the description**

- **JUSTIN S. LAFOUNTAINE et al.** Enabling thermal processing of ritonavir-polyvinyl alcohol amorphous solid dispersions by KinetiSol Dispersing. *European Journal of Pharmaceutics and Biopharmaceutics,* 2016, vol. 101, 72-81
- *J. Antibiotics,* 1985, vol. 38, 877-885
- *Chem. Pharm. Bull.,* 1990, vol. 38, 2792-2796